# EUROPEAN PATENT SPECIFICATION

(11) **EP 2 240 119 B1**
(45) Date of publication and mention of the grant of the patent: **28.03.2018**
(21) Application number: 08867792.7
(22) Date of filing: 18.12.2008
(51) Int. Cl.: A61F 2/07, A61F 2/89, A61F 2/91

(54) **STENT AND METHOD OF MAKING A STENT**
STENT UND VERFAHREN ZUR HERSTELLUNG EINES STENTS
STENT ET PROCÉDÉ DE FABRICATION D'UN STENT

(30) Priority: 26.12.2007 US 16737 P
(43) Date of publication of application: 20.10.2010
(73) Proprietor: Cook Medical Technologies LLC, Bloomington, IN 47404 (US)
(72) Inventor: DIERKING, William, K., Louisville, KY 40222 (US); LEEWOOD, Alan, R., Lafayette, IN 47905 (US); ROEDER, Blayne, A., Lafayette, IN 47909 (US)
(74) Representative: Jehan, Robert
(86) International application number: PCT/US2008/013863
(87) International publication number: WO 2009/085190

(56) References cited:
- EP-A- 0 539 237
- EP-A- 0 824 903
- US-A1- 2002 049 490

## Description

### Technical Field

This invention relates to endoluminal medical devices for implantation within the human or animal body for treatment of endovascular disease. In particular, this invention relates to stents for accommodating suture material having a locally polished region.

### Background of the invention

The functional vessels of human and animal bodies, such as blood vessels and ducts, occasionally weaken or even rupture. For example, the aortic wall can weaken, resulting in an aneurysm. One surgical intervention for weakened, aneurismal, or ruptured vessels involves the use of stent grafts to replace or repair the vessel. Stent grafts may be formed from a tube of a biocompatible material in combination with one or more stents to maintain a lumen therethrough. The stents are attached to the graft material in a number of ways, including by suturing the stent to the graft material.

It is preferable that these prostheses seal off the failed portion of the vessel. For weakened or aneurismal vessels, even a small leak in the prosthesis may lead to the pressurization of or flow in the treated vessel, which aggravates the condition the prosthesis was intended to treat. A prosthesis of this type can, for example, treat aneurysms of the abdominal aortic, iliac, or branch vessels such as the renal arteries.

The above-described examples are only some of the applications in which endoluminal devices are used by physicians. Many other applications for endoluminal devices are known and/or will be developed in the future. For example, in addition to the use of stents and stent-grafts to treat vascular stenosis and aneurysms, similar procedures may also be used to deploy vascular filters, occluders, artificial valves and other endoprosthetic devices.

In order to deliver a stent or stent-graft though narrow passageways, the stent is typically collapsed into a delivery configuration with a small diameter. The collapsed stent structure may then be inserted into a sheath which retains the stent in the delivery configuration until it is released. Because the stent must be significantly collapsed in this configuration, a large strain is introduced into the stent structure. Since a typical stent structure is only collapsed into the delivery configuration one time or a minimal number of times, it is generally considered that the stent structure can accommodate a large strain level in this application without resulting in permanent damage to the stent structure.

Once the stent is released at the site of implantation, the stent structure expands and contacts the lumen wall. In this process, a large portion of the strain is relieved. However, the stress of compression can cause damage to the stent-graft. Specifically, the stress of compression can cause the sutures to wear against the graft material.

The problem of suture wear is increased in diamond-shaped stents. Like other stents, the diamond-shaped stents may have eyelets to accommodate the sutures for suturing the stent to the graft. However, because these stents are so low profile, when they are compressed into the delivery device, they compress and leave no spaces. The edges of the eyelets thus wear on the suture and lead to unacceptable suture life span. They eventually fray and break.

Stent-grafts may also be subject to the problem of graft wear. Stents are often constructed by laser-cutting a cannula. Laser-cutting the cannula produced substantially rectangular, or even trapezoidal, stent cross-sections which can wear against the graft material and also cause the sutures to weaken. Additionally, at the regions where the stent contacts the graft, the graft material may weaken and tear due to the pressure of blood flow through the prosthesis. This graft wear contributes to a reduced life of the prosthesis.

Electropolishing methods may reduce the rough surfaces of the stent and decrease the blunt rectangular edges of the stent that often contribute to the problems of graft wear and suture wear. However, electropolishing tends to remove stent material in a relatively uniform manner. Therefore, electropolishing to remove material from the corners of the stent to create a more circular cross-section often results in stent material removed from the struts of the stent. The removal of material from the struts of the stent may result in a decreased integrity of the stent, reducing the overall life of the prosthesis.
US 2002/0049490 describes an endoluminal prosthesis having a compressible and expansible single-piece thick walled cylindrical structure. The cylindrical structure is comprised of curved elongated beams which intermittently merge with adjacent curved elongated beams. Each beam has a radial thickness greater than the circumferential width. The configuration of the curved beams reduces stress concentrations in the expanded and compressed condition of the prosthesis. Features are provided for high expansion ratios allowing the prosthesis to collapse into a very small diameter and expand into a very large diameter.

EP 0 539 237 describes a transluminal grafting system for grafting a prosthesis to the wall of a lumen. The system includes a tubular graft provided with spring assemblies and anchoring barbs. The prosthesis is mounted on an apertured tubular carrier and a central control means is inserted into the bore of the apertured carrier. Mooring loops are attached to the prosthesis, pass through the apertures of the tubular carrier, and engage the central control means. An introducer sheath covers the system for smooth insertion into a lumen. When the graft has been positioned, the central control means maintains the axial position of the prosthesis. When the introducer sheath is pulled, the prosthesis is exposed and the spring assemblies return to an expanded state and anchor the graft against the internal wall of the lumen.

### Summary of the invention

The present invention seeks to provide an improved endoluminal medical device, such as a stent and an improved method of manufacturing a medical device. The preferred embodiments provide an endoluminal device with an improved eyelet, where the eyelet region of the endoluminal device is locally polished. It has been found that the improved eyelet structure allows the graft material to be affixed to the stent without premature failure due to suture wear and/or graft wear.

According to an aspect of the present invention there is provided a stent for use in a stent graft as specified in claim 1.

The preferred embodiment provides a variably polished stent, in particular a stent with an improved eyelet, where the eyelet region of the stent is locally polished. A method of manufacturing the stent with an improved eyelet also is disclosed. The effect of locally polishing the eyelet region to yield rounded eyelet edges results in less stress to the material of the sutures and decreases graft wear, which increases the life of the overall endoluminal device.

The stent may include a strut region including at least two struts, the struts having at least one radius of curvature. A bend connects the two struts at an eyelet region. The strut region and the eyelet region are electropolished, and the eyelet region is locally polished. The eyelet positioned in the eyelet region has at least one radius of curvature that is greater than zero.

The struts have an edge having a radius of curvature. The radius of curvature of the struts approaches that of a sharp corner, which has a radius of curvature of zero (0). Because a perfectly sharp corner is not likely to be achieved after electropolishing, the radius of curvature of the strut may be less than .001 mm.

The eyelet region is locally polished, such that the edge of the eyelet becomes rounded. The radius of curvature of the eyelet will be approximately the same regardless of where along the edge of the eyelet the radius is measured. An acceptable range of the radius of curvature of the eyelet may be about 0.01 mm to a value where a cross-section of the eyelet is circular. For example, the radius of curvature of the may be limited no less than 1/10 of the radius of the suture that attaches the strut to graft material. In another example, the lower range of the radius of curvature of the eyelet may be at least an order of magnitude higher than the radius of curvature of the strut. In this example, the radius of curvature of the strut may be about 001 mm and the radius of curvature of the locally polished eyelet may be at eyelet may be limited to be no less than 1/10 of the radius of the suture, to avoid the eyelet severing or fraying the suture.

In another example, the lower range of the radius of curvature of the eyelet may be at least an order of magnitude higher than the radius of curvature of the strut. For example, the radius of curvature of the strut may be about 0.001 mm and the radius of curvature of the locally polished eyelet may be at least about 0.01 mm. In another example, the radius of curvature of the eyelet may be no less than 1/10 of the radius of the suture and is at least an order of magnitude greater than the at least one radius of curvature of the strut.

The stent may be formed from biocompatible material. The materials used in the manufacture of the device may be selected from a well-known list of suitable metals. Preferred materials include those materials that can provide the desired functional characteristics with respect to mechanical load bearing, biological compatibility, modulus of elasticity, or other desired properties. In various embodiments, the stent includes a metallic material selected from stainless steel, nickel, silver, platinum, palladium, gold, titanium, tantalum, iridium, tungsten, cobalt, chromium, a nickel-titanium alloy, a superelastic nickel-titanium (NiTi) alloy sold under the trade name NITINOL® or inconel. Preferably, the individual stent units are manufactured from Nitinol or stainless steel.

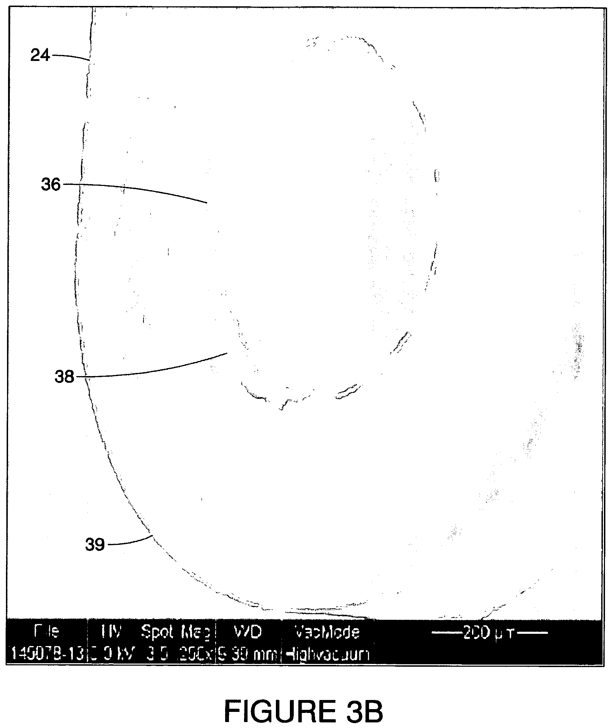

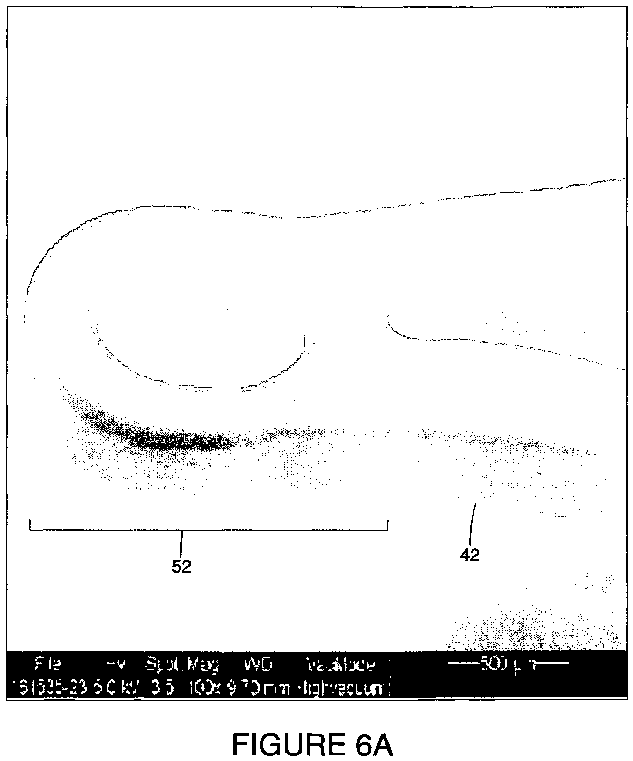

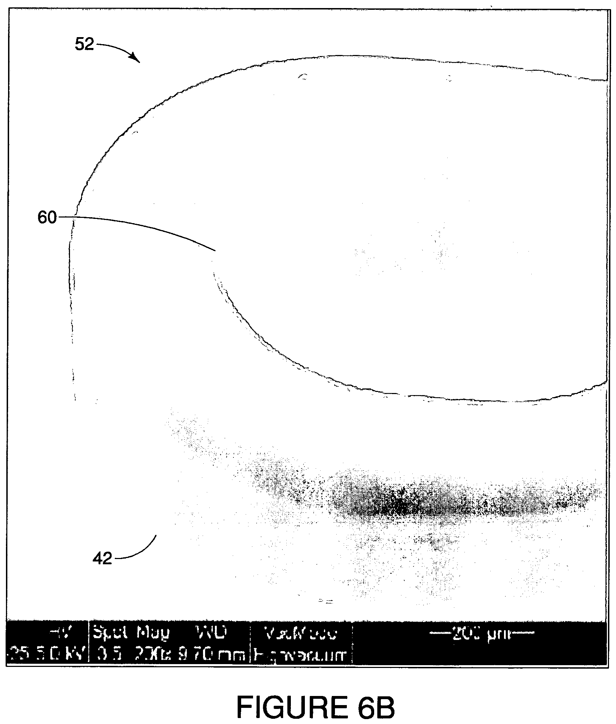

Figure 6A is an SEM image of the locally polished stent 42 which displays the eyelet region 52 at 100X magnification. The eyelet region 52 appears to be smooth. Figure 6B is an SEM image that displays the same locally polished stent 42 at 250X magnification and visually confirms the impact of the localized polishing on the eyelet region 52. The eyelet edges 60 are rounded and sharp edges 38 of the eyelet 36 of the electropolished stent 24 seen in Figure 3B are eliminated.

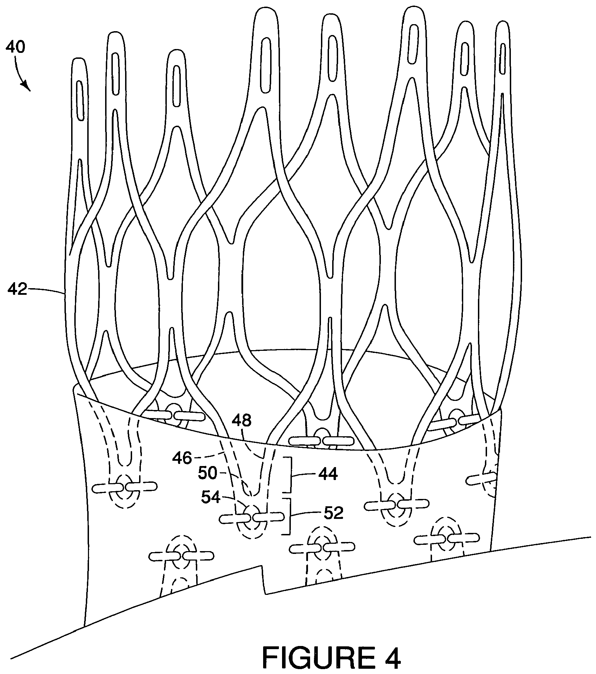

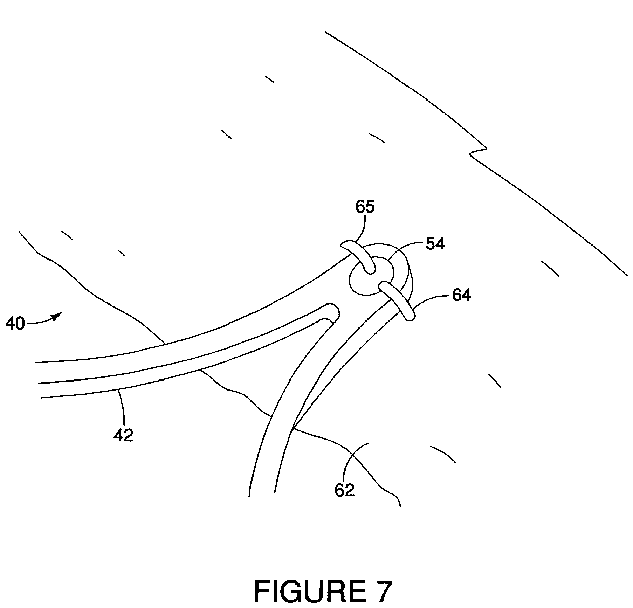

The locally polished stent 42 may be attached to graft material to form the endoluminal device 40 as shown in Figure 4. Figure 7 shows attachment of the locally polished stent 42 to graft material 62. For example, graft material 62 may be affixed to the stent 42 using sutures 64, 65 which are threaded through the eyelet 54 using a double suture technique. The eyelet may be an elliptical shape in order to accommodate the double suture attachment. Suture material may be polypropylene or any other suitable material known in the art.
as an artery. Tubular prosthetic devices include single and both branched and bifurcated devices.

The term "endoluminal" refers to or describes objects that can be placed inside a lumen or a body passageway in a human or animal body. A lumen or a body passageway can be an existing lumen or a lumen created by surgical intervention. As used in this specification, the terms "lumen" or "body passageway" are intended to have a broad meaning and encompasses any duct (e.g., natural or iatrogenic) within the human body and can include a member selected from the group comprising: blood vessels, respiratory ducts, gastrointestinal ducts, and the like. "Endoluminal device" or "endoluminal prosthesis" thus describes devices that can be placed inside one of these lumens.

The term "stent" means any device or structure that adds rigidity, expansion force or support to a prosthesis. A stent is used to obtain and maintain the patency of the body passageway while maintaining the integrity of the passageway. Also, the stent may be used to form a seal. The stent may be coated with a polymeric material, for example, by immersion in molten polymer or any other method known to one of skill in the art. A Z-stent is a stent that has alternating struts and peaks (*i.e*., bends) and defines a generally cylindrical lumen. The "amplitude" of a Z-stent is the distance between two bends connected by a single strut. The "period" of a Z-stent is the total number of bends in the Z-stent divided by two, or the total number of struts divided by two.

In one configuration, the stent may represent a plurality of discontinuous devices. In another configuration, the stent may represent one device. The stent may be located on the exterior of the device, the interior of the device, or both. A stent may be self-expanding, balloon-expandable or may have characteristics of both. A variety of other stent configurations are also contemplated by the use of the term "stent".

The term "graft" or "graft material" describes an object, device, or structure that is joined to or that is capable of being joined to a body part to enhance, repair, or replace a portion or a function of that body part. A graft by itself or with the addition of other elements, such as structural components, can be an endoluminal prosthesis. The graft comprises a single material, a blend of materials, a weave, a laminate, or a composite of two or more materials. The graft can also comprise polymer material that may be layered onto the mandrel of the described embodiments. Preferably, polymers, although added in layers onto the mandrel, after curing, result in one layer that encapsulates a stent or woven graft. This also aids in decreasing the incidence of delamination of the resulting endovascular prosthesis. A stent may be attached to a graft to form a "stent graft."

The term "patient" as used in this application refers to any mammal, especially humans.

The teachings herein provide an endoluminal device with an improved eyelet, where the eyelet region of the implantable device is locally polished. In another aspect, the teachings herein provide for an eyelet geometry which is non-circular, such as oval, elliptical, slot-shaped and the like. A method of manufacturing the endoluminal device with an improved eyelet also is described.

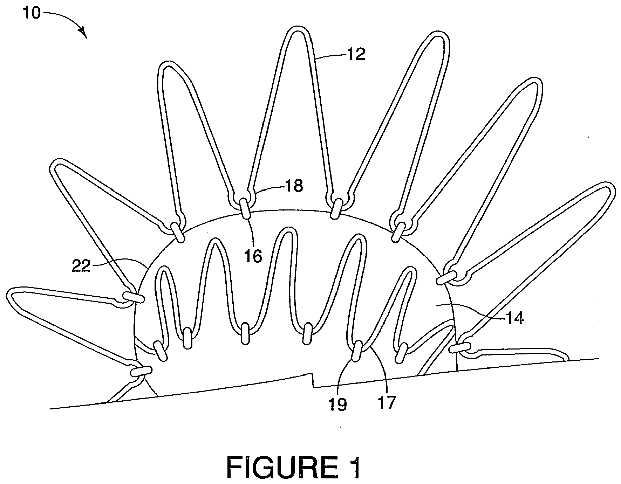

Referring now to the drawings, and particularly to Figure 1, a conventional endoluminal device 10 is shown. The implantable device 10 is formed of an attachment z-stent 12 that is secured to main body 14 of the implantable device by threading a suture 16 through the loops 18 at the end of the attachment z-stent 12 and connecting the attachment z-stent 12 to the graft material 22. Proximal sealing stent 17 is attached to the graft material 22 via a suture 19.

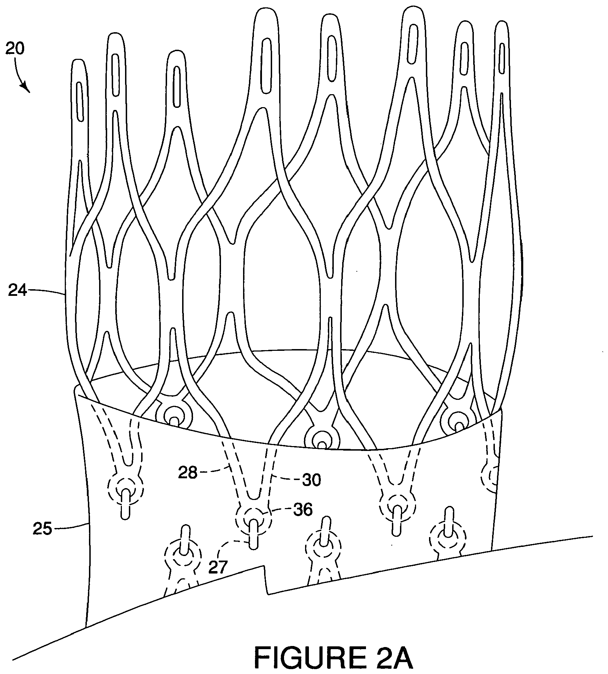

Figure 2A shows an implantable device 20 comprising a stent 24, specifically a cannula cut diamond-shaped stent, in which the entire stent 24 has been electropolished. This implantable device 20 provides significant radial force, but maintains a low profile in its loaded configuration. An example of such a diamond shaped stent device is described in US-A- 2007/0021824 entitled "Endoluminal Device With Improved Tapered Beams," which is herein incorporated by reference.

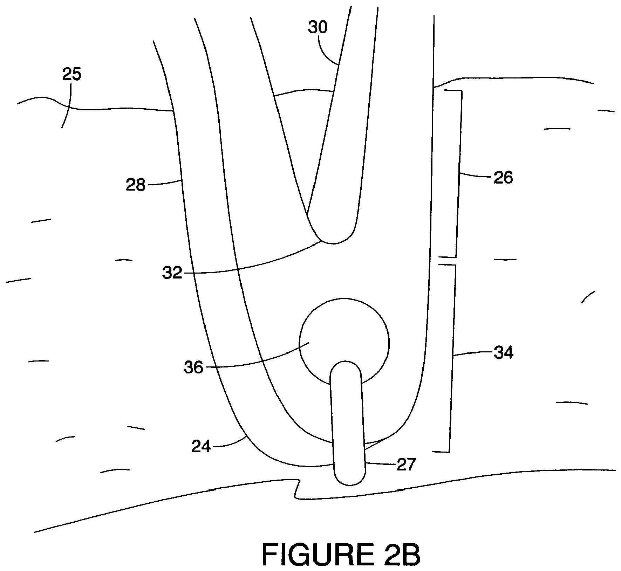

Figure 2B depicts an enlarged view of the stent 24. The stent 24 includes a strut region 26, having at least two struts 28, 30 that are connected by a bend 32 at an eyelet region 34. The strut region 26 is the main "load carrying" portion of the stent which may be laser cut and electropolished. An eyelet 36, which may be substantially circular, is located at the eyelet region 34. The stent 24 is connected to graft material 25 via an eyelet 36 with a suture 27.

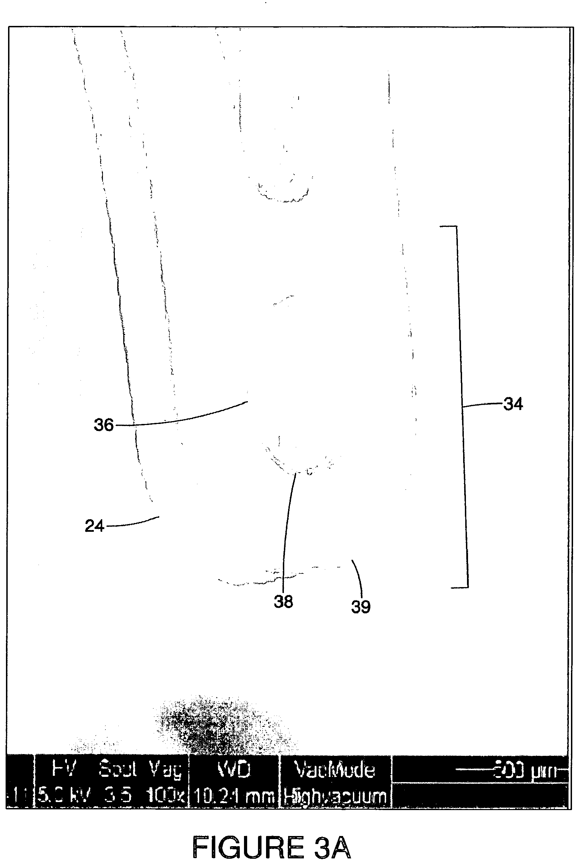

The nature of any cannula cut stent is that edges are created due to the substantially rectangular or trapezoidal cross-section as a result of the laser cutting process. Even though these stents are electropolished to evenly remove material, a relatively sharp edge remains. Figure 3A, an SEM image of the an electropolished cannula cut stent 24, displays an eyelet region 34 at 100X magnification. The eyelet region 34 appears to be smooth. However, Figure 3B is an SEM image that displays the same electropolished cannula cut stent 24 at 250X magnification. Figure 3B reveals that the edges 38, 39 of the eyelet 36 are sharp and that the cross-section of the eyelet 36 is substantially rectangular.

Figure 4 shows an endoluminal device 40 comprising a stent 42, specifically a cannula cut diamond-shaped stent. This endoluminal device 40 provides significant radial force, but maintains a low profile in its loaded configuration. The stent 42 is comprised of at strut region 44 having at least two struts 46, 48 that are connected by a bend 50 at an eyelet region 52. The strut region 44 is the main "load carrying" portion of the stent 42. The stent 42 is electropolished. An eyelet 54 is located at the eyelet region 52. In this stent 42, in contrast to the stent 24 discussed above, the eyelet region 52 has been locally polished to smooth out the sharp edges of the eyelet 54.

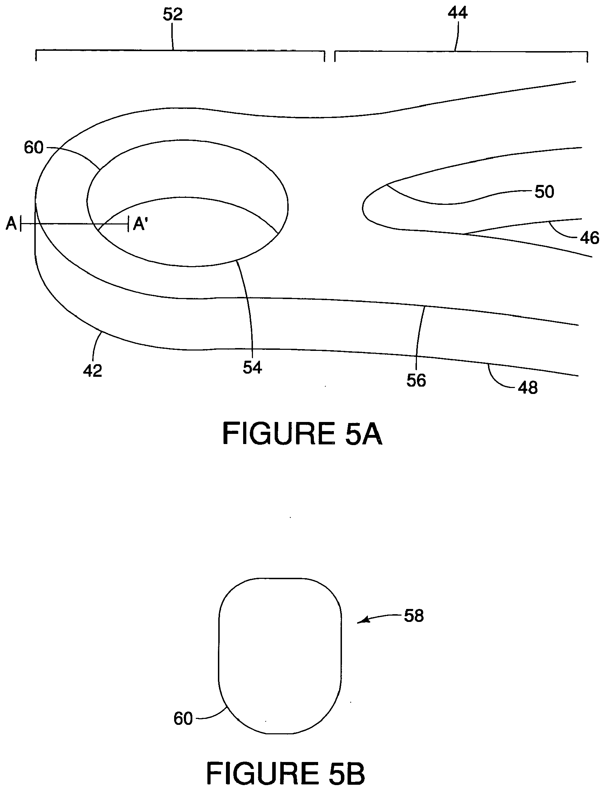

Referring to Figure 5A, an enlarged view of the stent 42, displays the properties of the locally polished eyelet region 52. The strut 48 has an edge 56 having a radius of curvature. The radius of curvature of the strut approaches that of a sharp corner, which has a radius of curvature of zero (0). Because a perfectly sharp corner is not likely to be achieved after electropolishing, the radius of curvature of the strut is typically less than 0.001 mm. This value is adequate to maintain a strut 48 cross-section sufficient to achieve adequate durability and radial force.

As the eyelet region 52 is locally polished, the edge 60 of the eyelet 54 will become more rounded and the cross-section of the eyelet 58 will become more circular. Figure 5B depicts the eyelet cross-section 58 taken along the line A-A'. The radius of curvature of the eyelet will be approximately the same regardless of where along the edge 60 of the eyelet 54 the radius is measured. In one example, an acceptable range of the radius of curvature of the eyelet is about 0.01 mm to a value where a cross-section of the eyelet 54 is circular. For example, the radius of curvature of the eyelet may be 0.10 mm. In addition, the radius of curvature of the eyelet may be limited to be no less than 1/10 of the radius of the suture, to avoid the eyelet severing or fraying the suture.

In another example, the lower range of the radius of curvature of the eyelet may be at least an order of magnitude higher than the radius of curvature of the strut. For example, the radius of curvature of the strut may be about 0.001 mm and the radius of curvature of the locally polished eyelet may be at least about 0.01 mm. In another example, the radius one radius of curvature of the eyelet may be no less than 1/10 radius of suture and is at least an order of magnitude greater than the at least one radius of curvature of the strut.

The stent may be formed from biocompatible material. The materials used in the manufacture of the device may be selected from a well-known list of suitable metals. Preferred materials include those materials that can provide the desired functional characteristics with respect to mechanical load bearing, biological compatibility, modulus of elasticity, or other desired properties. In various embodiments, the stent includes a metallic material selected from stainless steel, nickel, silver, platinum, palladium, gold, titanium, tantalum, iridium, tungsten, cobalt, chromium, a nickel-titanium alloy, a superelastic nickel-titanium (NiTi) alloy sold under the trade name NITINOL® or inconel. Preferably, the individual stent units are manufactured from Nitinol or stainless steel.

Figure 6A is an SEM image of the locally polished stent 42 which displays the eyelet region 52 at 100X magnification. The eyelet region 52 appears to be smooth. Figure 6B is an SEM image that displays the same locally polished stent 42 at 250X magnification and visually confirms the impact of the localized polishing on the eyelet region 52. The eyelet edges 60 are rounded and sharp edges 38 of the eyelet 36 of the electropolished stent 24 seen in Figure 3B are eliminated.

The locally polished stent 42 may be attached to graft material to form the endoluminal device 40 as shown in Figure 4. Figure 7 shows attachment of the locally polished stent 42 to graft material 62. For example, graft material 62 may be affixed to the stent 42 using sutures 64, 65 which are threaded through the eyelet 54 using a double suture technique. The eyelet may be an elliptical shape in order to accommodate the double suture attachment. Suture material may be polypropylene or any other suitable material known in the art.

The tubular graft material may be constructed from a biocompatible textile fabric, a polymer, biomaterial, or a composite thereof. Examples of biocompatible materials from which textile graft material can be formed include polyesters, such as polyethylene terephthalate); fluorinated polymers, such as polytetrafluoroethylene (PTFE) and fibers of expanded PTFE; and polyurethanes. Preferably, the graft material is a woven polyester. More preferably, the graft material is a polyethylene terephthalate (PET), such as DACRON® (DUPONT, Wilmington, DE) or TWILLWEAVE MICREL® (VASCUTEK, Renfrewshire, Scotland). Woven polyesters, such as Dacron, possess varying degrees of porosity, where the degree of porosity can be selectively controlled based on the weaving or knitting process that is used to produce the woven polyester. Consequently, depending on the application, the porosity can be adjusted to encourage incorporation of a patient's tissue into the woven graft material, which in turn may more securely anchor the prosthesis within the patient's vessel or lumen. Furthermore, the degree of porosity can also be adjusted to provide a woven graft material that is impermeable to liquids, including blood or other physiological fluids. The woven polyester of the graft material may comprise a plurality of yarns.

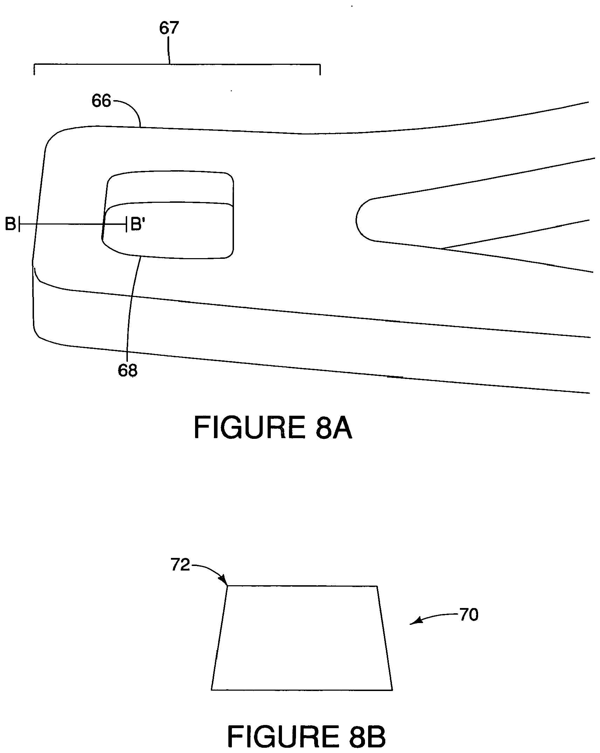

A method of manufacturing the implantable device 40 also is provided. Standard laser cutting techniques which are known in the art may be employed to manufacture the stent 42 as described above. For example, the stent structure may be fabricated by laser cutting the structural members from a tube. Figure 8A displays an enlarged view of the eyelet region 67 of the stent 66 after it is laser cut. The eyelet of the stent 68 also is laser cut. A cross-section 70 of the eyelet 68 taken along the line B-B' is shown in Figure 8B. As shown in Figure 8B, the shape of the eyelet after it is laser cut is trapezoidal and the edge 72 is a substantially sharp corner.

The entire stent device may then be electropolished. Electropolishing is the electrolytic removal of a metal in a preferably highly ionic solution by means of electrical potential and current. Electropolishing is preferably used to smooth, polish, de-burr or clean an electrically conductive material. It removes stress concentrations by selectively removing surface defects on metal surfaces, thereby making the material stronger. Electropolishing can also improve corrosion resistance and remove hydrogen from the surface of the stent.

Electropolishing typically involves providing an electrolytic solution, placing the stent within the electrolytic solution, placing a cathode within the solution and not contacting the stent and coupling an anode to the stent. When an electric voltage is provided between the anode and the cathode, the stent is caused to lose portions of its outer surface when the elements forming the stent are driven into solution and carried to the cathode for deposition upon the cathode. The rougher surfaces of the stent are more readily driven into solution and hence removed from the surfaces of the stent, smoothing the surfaces of the stent somewhat.

The electropolishing process often begins with the preparation of the stent by cleaning it, which can remove non-conductive material from the surface of the stent. Oils, glues and other substances are possible contaminants. Then, the stent can be electropolished by placing it in an acid bath, preferably a phosphoric and sulfuric acid solution, and connecting the positive lead of a DC power supply to the stent and a negative lead to a cathode. Post-treatment preferably involves placing the stent in a nitric acid rinse followed by a water rinse. Figure 2B and Figures 3A-3B depict a stent device following the step of electropolishing.

The eyelet region is locally polished according to the dimensions discussed in above. The local polishing may include mechanical tumbling or polishing to smooth the blunt eyelet edges, followed by electropolishing according to methods known in the art. In one example, this local polishing is done in a subsequent step to the electropolishing of the entire stent. Figures 6A-6B display SEM images of the stent 42 after the eyelet region has been locally polished. Other regions of the stent, such as the mid-strut regions, where it is desired to selectively remove stent material in order decrease graft wear, may also be locally polished.

As shown in Figure 7, the locally polished stent 42 may then be attached to graft material 62. In one example, the graft material 62 is affixed to the stent 42 using sutures 64 which are threaded through the eyelet 54. The suture may be threaded twice through the eyelet using a double suture technique. In this example, the eyelet is an elliptical shape in order to accommodate the double suture attachment. The endoluminal device 40 is formed of at least one locally polished stent 42 attached to graft material 62.

Other shapes of eyelet may be used in place of elliptical, including oval, slot-shaped and similar shapes.

The endoluminal device 40 is delivered and positioned in the body vessel using methods known in the art. For example, the device may be mounted within a retaining sheath which contacts the outer surface of the stent and retains the stent in a compressed state for delivery into a vessel. A hollow needle may be used to penetrate the vessel, and a guide wire may be threaded through the needle into the vessel. The needle may then be removed and replaced with an introduction catheter, which generally acts as a port through which endoluminal devices, including stents, may then be passed to gain access to a vessel. The compressed stent and the retaining sheath may then be passed through the introduction catheter into the vessel. Once the stent is positioned within the vessel adjacent to the site to be treated, the retaining sheath may be retracted, thereby causing the stent to expand from the compressed state to an expanded state. In the expanded state, the stent contacts and exerts a radial force on the vessel wall. The retaining sheath and the introduction catheter may then be withdrawn from the vessel.

It has been found that certain eyelet geometries, particularly elliptical, oval and slot-shaped, can reduce the relative movement of the suture across the eyelet perimeter as well as interfacial pressure which in turn can reduce suture wear. Such geometries can reduce wear as a result of reduction in suture diameter, number of sutures and suture loading.

It is therefore intended that the foregoing detailed description be regarded as illustrative rather than limiting, and that it be understood that it is the following claims that are intended to define the scope of this invention.

## Claims

1. A stent (42) for use in a stent graft including:
a strut region (44) comprising at least two struts (46,48), each strut having an edge (56) having a radius of curvature;
a bend (50) connecting the at least two struts and forming an eyelet region (52); and
an eyelet (54) positioned in the eyelet region (52), **characterised by** said eyelet (52) having a rounded edge (60) having a radius of curvature which is at least an order of magnitude greater than the radius of curvature of the edge (56) of the at least two struts (46,48).

2. A stent according to claim 1, wherein the radius of curvature of the edge (56) of the at least two struts (46,48) is about 0.001 mm or less.

3. A stent according to claim 1, where the radius of curvature of the edge (60) of the eyelet (54) is 0.01 mm and the radius of curvature of the edge (56) of the at least two struts (46,48) is 0.001 mm.

4. A stent according to any preceding claim, wherein the radius of curvature of the edge (60) of the eyelet (54) is in a range of about 0.01 mm to a value where a cross-section of the eyelet is circular.

5. A stent according to any preceding claim, wherein the radius of curvature of the edge (60) of the eyelet (54) is 0.10mm.

6. A stent according to any preceding claim, wherein the eyelet (54) has a non circular shape and is generally elliptical, oval or slot shaped.

7. A stent according to any preceding claim, wherein the radius of curvature of the edge (60) of the eyelet (54) is no less than 1/10 of the radius of at least one suture and is at least an order of magnitude greater than the radius of curvature of the edge (56) of the at least two struts (46,48).

8. A stent according to claim 1, comprising at least two or more of any of the following:
the radius of curvature of the edge (56) of the at least two struts (46,48) is about 0.001 mm or less;
the eyelet (54) has a generally elliptical shape;
the radius of curvature of the edge (60) of eyelet (54) is in a range of about 0.01 mm to a value where a cross-section of the eyelet is circular;
the radius of curvature of the edge (60) of the eyelet (54) is 0.01 mm and the radius of curvature of the at least two struts (46,48) is 0.001 mm;
the radius of curvature of the edge (60) of the eyelet (54) is no less than 1/10 a radius of at least one suture;
the radius of curvature of the edge (60) of the eyelet (54) is no less than 1/10 radius of at least one suture and is at least an order of magnitude greater than the radius of curvature of the edge (56) of the at least two struts (46,48).

9. A stent according to any preceding claim, wherein the eyelet (54) is secured to a graft material with at least one suture.

10. A stent according to claim 9, where the eyelet (54) is secured to the graft material with two sutures.

11. A stent according to claim 9 or 10, wherein the radius of curvature of the edge (64) of the eyelet (54) is no less than 1/10 a radius of at least one suture.

12. A method of manufacturing a stent (42), including the steps of:
laser cutting a cannula of stent material to form the stent (42), the stent comprising a strut region (44) comprising at least two struts (46,48), the at least two struts (46,48) having an edge (56) having a radius of curvature, and an eyelet region (52), the eyelet region (52) comprising an eyelet (54) having an edge (60) having a radius of curvature;
electropolishing the stent (52) and the eyelet region (52); and
selectively polishing the eyelet region (52) such that the at least one radius of curvature of the eyelet (54) at least an order of magnitude greater than the radius of curvature of the edge (56) of the at least two struts (46,48).

13. A method according to claim 12, including selectively polishing the eyelet region (52) such that the radius of curvature of the edge (60) of the eyelet (54) is in a range of about 0.01 mm to a value where a cross-section of the eyelet (54) is circular.

14. A method according to claim 12 or 13, including selectively polishing the eyelet region (52) such that the radius of curvature of the edge (60) of the eyelet (54) is 0.10mm.

15. A method according to claim 12, 13 or 14, including selectively polishing the eyelet region (52) such that the radius of curvature of the edge (60) of the eyelet (54) is no less than 1/10 the radius of at least one suture.

16. A method according to any one of claims 12 to 15, including securing the eyelet (54) to a graft material with at least one suture.

## Patentansprüche

1. Stent (42) zur Verwendung in einem Stenttransplantat, umfassend:
einen Strebenbereich (44), der wenigstens zwei Streben (46, 48) umfasst, wobei jede Strebe einen Rand (56) mit einem Krümmungsradius aufweist;
eine Biegung (50), die die wenigstens zwei Streben verbindet und einen Ösenbereich (52) bildet; und
eine Öse (54), die in dem Ösenbereich (52) angeordnet ist, **dadurch gekennzeichnet, dass** die Öse (52) einen abgerundeten Rand (60) mit einem Krümmungsradius, der wenigstens um eine Größenordnung größer als der Krümmungsradius des Rands (56) der wenigstens zwei Streben (46, 48) ist, aufweist.

2. Stent gemäß Anspruch 1, wobei der Krümmungsradius des Rands (56) der wenigstens zwei Streben (46, 48) etwa 0,001 mm oder weniger beträgt.

3. Stent gemäß Anspruch 1, wobei der Krümmungsradius des Rands (60) der Öse (54) 0,01 mm beträgt und der Krümmungsradius des Rands (56) der wenigstens zwei Streben (46, 48) 0,001 mm beträgt.

4. Stent gemäß einem der vorstehenden Ansprüche, wobei der Krümmungsradius des Rands (60) der Öse (54) in einem Bereich von etwa 0,01 mm bis zu einem Wert, bei dem der Querschnitt der Öse kreisförmig ist, liegt.

5. Stent gemäß einem der vorstehenden Ansprüche, wobei der Krümmungsradius des Rands (60) der Öse (54) 0,10 mm beträgt.

6. Stent gemäß einem der vorstehenden Ansprüche, wobei die Öse (54) eine nicht kreisförmige Form aufweist und allgemein elliptisch, oval oder schlitzförmig ist.

7. Stent gemäß einem der vorstehenden Ansprüche, wobei der Krümmungsradius des Rands (60) der Öse (54) nicht weniger als 1/10 des Radius wenigstens eines Nahtfadens beträgt und um wenigstens eine Größenordnung größer als der Krümmungsradius des Rands (56) der wenigstens zwei Streben (46, 48) ist.

8. Stent gemäß Anspruch 1, umfassend wenigstens zwei oder mehr von beliebigen von Folgenden:
der Krümmungsradius des Rands (56) der wenigstens zwei Streben (46, 48) beträgt etwa 0,001 mm oder weniger;
die Öse (54) weist eine allgemein elliptische Form auf;
der Krümmungsradius des Rands (60) der Öse (54) liegt in einem Bereich von etwa 0,01 mm bis zu einem Wert, bei dem der Querschnitt der Öse kreisförmig ist;
der Krümmungsradius des Rands (60) der Öse (54) beträgt 0,01 mm und der Krümmungsradius der wenigstens zwei Streben (46, 48) beträgt 0,001 mm;
der Krümmungsradius des Rands (60) der Öse (54) beträgt nicht weniger als 1/10 des Radius wenigstens eines Nahtfadens;
der Krümmungsradius des Rands (60) der Öse (54) beträgt nicht weniger als 1/10 des Radius wenigstens eines Nahtfadens und ist um wenigstens eine Größenordnung größer als der Krümmungsradius des Rands (56) der wenigstens zwei Streben (46, 48).

9. Stent gemäß einem der vorstehenden Ansprüche, wobei die Öse (54) mit wenigstens einem Nahtfaden an einem Transplantatmaterial befestigt ist.

10. Stent gemäß Anspruch 9, wobei die Öse (54) mit zwei Nahtfäden an dem Transplantatmaterial befestigt ist.

11. Stent gemäß Anspruch 9 oder 10, wobei der Krümmungsradius des Rands (64) der Öse (54) nicht weniger als 1/10 des Radius wenigstens eines Nahtfadens beträgt.

12. Verfahren zum Herstellen eines Stents (42), umfassend die Schritte:
Laserschneiden einer Kanüle aus Stentmaterial, um den Stent (42) zu bilden, wobei der Stent einen Strebenbereich (44), der wenigstens zwei Streben (46, 48) umfasst, wobei die wenigstens zwei Streben (46, 48) einen Rand (56) mit einem Krümmungsradius aufweisen, und einen Ösenbereich (52) umfasst, wobei der Ösenbereich (52) eine Öse (54) umfasst, die einen Rand (60) mit einem Krümmungsradius aufweist;
Elektropolieren des Stents (52) und des Ösenbereichs (52); und
selektives Polieren des Ösenbereichs (52), so dass der wenigstens eine Krümmungsradius der Öse (54) um wenigstens eine Größenordnung größer als der Krümmungsradius des Rands (56) der wenigstens zwei Streben (46, 48) ist.

13. Verfahren gemäß Anspruch 12, umfassend selektives Polieren des Ösenbereichs (52), so dass der Krümmungsradius des Rands (60) der Öse (54) in einem Bereich von etwa 0,01 mm bis zu einem Wert, bei dem der Querschnitt der Öse (54) kreisförmig ist, liegt.

14. Verfahren gemäß Anspruch 12 oder 13, umfassend selektives Polieren des Ösenbereichs (52), so dass der Krümmungsradius des Rands (60) der Öse (54) 0,10 mm beträgt.

15. Verfahren gemäß Anspruch 12, 13 oder 14, umfassend selektives Polieren des Ösenbereichs (52), so dass der Krümmungsradius des Rands (60) der Öse (54) nicht weniger als 1/10 des Radius wenigstens eines Nahtfadens beträgt.

16. Verfahren gemäß wenigstens einem der Ansprüche 12 bis 15, umfassend Befestigen der Öse (54) an einem Transplantatmaterial mit wenigstens einem Nahtfaden.

## Revendications

1. Endoprothèse (42) vasculaire à utiliser dans un greffon d'endoprothèse comprenant :
une zone (44) de traverse comprenant au moins deux traverses (46, 48), chaque traverse comportant un bord (56) ayant un rayon de courbure ;
un coude (50) raccordant lesdites traverses et constituant une zone (52) d'oeillet ; et
un oeillet (54) situé dans la zone (52) d'oeillet, **caractérisé en ce que** ledit oeillet (52) comporte un bord (60) arrondi ayant un rayon de courbure qui est au moins un ordre de grandeur supérieur au rayon de courbure du bord (56) desdites traverses (46, 48).

2. Endoprothèse selon la revendication 1, dans laquelle le rayon de courbure du bord (56) desdites traverses (46, 48) est d'environ 0,001 mm ou moins.

3. Endoprothèse selon la revendication 1, dans laquelle le rayon de courbure du bord (60) de l'oeillet (54) est de 0,01 mm et le rayon de courbure du bord (56) desdites traverses (46, 48) est de 0,001 mm.

4. Endoprothèse selon l'une quelconque des revendications précédentes, dans laquelle le rayon de courbure du bord (60) de l'oeillet (54) est dans une plage allant d'environ 0,01 mm à une valeur où la section transversale de l'oeillet est circulaire.

5. Endoprothèse selon l'une quelconque des revendications précédentes, dans laquelle le rayon de courbure du bord (60) de l'oeillet (54) est de 0,10 mm.

6. Endoprothèse selon l'une quelconque des revendications précédentes, dans laquelle l'oeillet (54) a une forme non circulaire et est dans l'ensemble elliptique, ovale ou en forme de fente.

7. Endoprothèse selon l'une quelconque des revendications précédentes, dans laquelle le rayon de courbure du bord (60) de l'oeillet (54) est supérieur ou égal au 1/10^{e} du rayon d'au moins une suture et il est au moins un ordre de grandeur supérieur au rayon de courbure du bord (56) desdites traverses (46, 48).

8. Endoprothèse selon la revendication 1, comprenant au moins deux ou plusieurs caractéristiques parmi toutes les suivantes :
le rayon de courbure du bord (56) desdites traverses (46, 48) est d'environ 0,001 mm ou moins ;
l'oeillet (54) a une forme dans l'ensemble elliptique ;
le rayon de courbure du bord (60) de l'oeillet (54) est dans une plage allant d'environ 0,01 mm à une valeur où la section transversale de l'oeillet est circulaire ;
le rayon de courbure du bord (60) de l'oeillet (54) est de 0,01 mm et le rayon de courbure desdites traverses (46, 48) est de 0,001 mm ;
le rayon de courbure du bord (60) de l'oeillet (54) est supérieur ou égal au 1/10^{e} du rayon d'au moins une suture ;
le rayon de courbure du bord (60) de l'oeillet (54) est supérieur ou égal au 1/10^{e} du rayon d'au moins une suture et il est au moins un ordre de grandeur supérieur au rayon de courbure du bord (56) desdites traverses (46, 48).

9. Endoprothèse selon l'une quelconque des revendications précédentes, dans laquelle l'oeillet (54) est solidement fixé à un matériau de greffon par au moins une suture.

10. Endoprothèse selon la revendication 9, dans laquelle l'oeillet (54) est solidement fixé au matériau de greffon par deux sutures.

11. Endoprothèse selon la revendication 9 ou 10, dans laquelle le rayon de courbure du bord (64) de l'oeillet (54) est supérieur ou égal au 1/10^{e} du rayon d'au moins une suture.

12. Procédé de fabrication d'une endoprothèse (42) comprenant les étapes consistant à :
découper par laser une canule de matériau d'endoprothèse pour constituer l'endoprothèse (42), l'endoprothèse comprenant une zone (44) de traverse comprenant au moins deux traverses (46, 48), lesdites traverses (46, 48) comportant un bord (56) ayant un rayon de courbure, et une zone (52) d'oeillet, la zone (52) d'oeillet comprenant un oeillet (54) comportant un bord (60) ayant un rayon de courbure ;
polir par électrolyse l'endoprothèse (52) et la zone (52) d'oeillet ; et
polir sélectivement la zone (52) d'oeillet de telle sorte que ledit rayon de courbure de l'oeillet (54) est au moins un ordre de grandeur supérieur au rayon de courbure du bord (56) desdites traverses (46, 48).

13. Procédé selon la revendication 12, comprenant l'opération consistant à polir sélectivement la zone (52) d'oeillet de telle sorte que le rayon de courbure du bord (60) de l'oeillet (54) est dans une plage allant d'environ 0,01 mm à une valeur où la section transversale de l'oeillet (54) est circulaire.

14. Procédé selon la revendication 12 ou 13, comprenant l'opération consistant à polir sélectivement la zone (52) d'oeillet de telle sorte que le rayon de courbure du bord (60) de l'oeillet (54) est de 0,10 mm.

15. Procédé selon l'une quelconque des revendications 12, 13 ou 14, comprenant l'opération consistant à polir sélectivement la zone (52) d'oeillet de telle sorte que le rayon de courbure du bord (60) de l'oeillet (54) est supérieur ou égal au 1/10^{e} du rayon d'au moins une suture.

16. Procédé selon l'une quelconque des revendications 12 à 15, comprenant l'opération consistant à solidement fixer l'oeillet (54) à un matériau de greffon par au moins une suture.
